# FASCICULE DE BREVET EUROPEEN

(11) **EP 0 605 646 B1**
(45) Date de publication et mention de la délivrance du brevet: **08.07.1998**
(21) Numéro de dépôt: 92921505.1
(22) Date de dépôt: 25.09.1992
(51) Int. Cl.: A61K 31/47, A61K 31/495, A61K 9/08, A61K 47/12

(54) **SELS DE SPARFLOXACINE ET SOLUTIONS LES CONTENANT**
Sparfloxacin-Salze und sie enthaltende Lösungen
SPARFLOXACINE SALTS AND SOLUTIONS CONTAINING SAME

(30) Priorité: 27.09.1991 FR 9111913
(43) Date de publication de la demande: 13.07.1994
(73) Titulaire: RHONE-DPC EUROPE, 92160 Antony (FR)
(72) Inventeur: CONRATH, Guillaume, F-92160 Antony (FR)
(74) Mandataire: Savina, Jacques
(86) Numéro de dépôt international: FR9200891
(87) Numéro de publication internationale: WO9305782

(56) Documents cités:
- EP-A- 219 784
- EP-A- 221 463
- EP-A- 284 935
- EP-A- 322 892
- J. of Parenteral Science & Technology, vol. 40, No. 2, 1986, p. 70-72, Carola H. Spurlock, Increasing Solubility of Enoxacin and Norfloxacin by Means of Salt Formation.

## Description

La présente invention concerne une nouvelle composition pharmaceutique convenable pour l'administration parentérale de la sparfloxacine.

Dans la demande de brevet EP 221 463 a été décrite la sparfloxacine de formule : 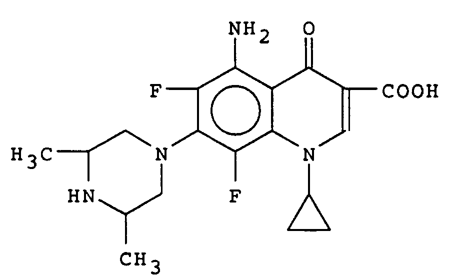 ainsi que ses sels d'addition avec des acides ou des bases, utile comme agent antimicrobien.

La demande de brevet EP 284935 décrit aussi des quinolones et leurs sels et parmi ces produits, la sparfloxacine.

Malheureusement les sels de la sparfloxacine sont généralement insolubles ou instables en solution. Il n'avait donc pas été possible jusgu'à présent de préparer des compositions convenables pour l'administration parentérale.

Le brevet européen EP 322 892 mentionne ces difficultés et décrit des compositions lyophilisées contenant un sel de la sparfloxacine avec un acide. Néanmoins ces compositions ne permettent pas d'obtenir des solutions stables pendant un temps prolongé et de ce fait seule la mise en solution extemporanée est possible. De plus de telles solutions risquent d'être impropres à un usage en perfusions lentes.

La présente invention concerne des solutions injectables stables. Ces compositions peuvent être conservées pendant un temps prolongé et sont en général stables à la chaleur.

Les solutions selon l'invention sont des solutions aqueuses contenant
- la sparfloxacine,
- un ou plusieurs polyhydroxyacides monocarboxyliques ou leurs dérivés lactone, au moins en quantité stoechiométrique par rapport à la sparfloxacine,
- si nécessaire un excès du polyhydroxyacide monocarboxylique ou d'un autre acide pharmaceutiquement acceptable destiné à assurer un pH de solubilisation complète du sel ainsi formé, inférieur ou égal à 5,
- éventuellement un agent isotonisant et/ou d'autres adjuvants pharmaceutiquement acceptables.

Les solutions selon l'invention contiennent au moins 1% de sparfloxacine. Elles peuvent néanmoins contenir des concentrations pouvant aller jusqu'à 40% de sparfloxacine. Il est bien entendu que des solutions de concentration inférieure à 1% sont également réalisables et cliniquement utilisables ; ces solutions entrent également dans le cadre de la présente invention.

Le polyhydroxyacide monocarboxylique est choisi de telle manière que son pK_{A} soit supérieur à 3, à 25°C. A titre d'exemple il peut être choisi parmi les acides lactobionique, glucoheptonique, gluconique ou ascorbique.

La quantité du polyhydroxyacide monocarboxylique est fonction de la quantité de sparfloxacine. Elle est déterminée de manière à avoir au moins les proportions stoechiométriques et de telle manière à obtenir de préférence des solutions dont le pH n'est pas inférieur à 3,5.

L'acide capable d'assurer une solubilisation complète peut être choisi parmi les acides pharmaceutiquement acceptables qui ne risquent pas, de par leur nature et/ou leur concentration, de déplacer de façon importante le sel de la sparfloxacine avec le polyhydroxyacide monocarboxylique. A titre d'exemple peuvent être choisis des acides organiques comme l'acide acétique, l'acide propionique, l'acide tartrique, l'acide succinique ou les hydroxy ou polyhydroxyacides monocarboxyliques. Des acides plus forts peuvent également être utilisés comme notamment les acides sulfoniques (acide méthanesulfonique par exemple), les acides carboxyliques (acide maléique, acide oxalique, acide malonique par exemple), ou les acides minéraux (par exemple acide chlorhydrique, acide phosphorique, acide sulfurique) dans les cas où leur concentration pourra être fixée de telle manière qu'ils ne risquent pas de déplacer de façon importante le sel de la sparfloxacine avec le polyhydroxyacide monocarboxylique. Il est néanmoins entendu que pour des questions de commodité, il est tout à fait avantageux d'ajuster la solubilité et le pH des solutions au moyen du polyhydroxyacide monocarboxylique employé.

Le pH des solutions est inférieur ou égal à 5. D'une manière générale le pH des solutions doit être compatible avec une administration directe. De préférence il est compris entre 3,5 et 5.

De préférence les solutions selon l'invention sont isotoniques. Néanmoins les solutions non isotoniques destinées à une injection dans une poche de perfusion au glucose entrent également dans le cadre de la présente invention.

Les solutions selon l'invention peuvent être isotonisées par addition d'un agent isotonisant tel que par exemple le glucose, le glycérol, le sorbitol, le mannitol, le xylitol, le fructose, ou le lactose.

Les solutions selon l'invention sont plus spécialement destinées à l'administration par voie parentérale. Elles peuvent être également utilisées par voie orale, occulaire, auriculaire ou en application locale sur la peau et les muqueuses.

Il est entendu que les solutions selon l'invention peuvent contenir, outre l'isotonisant, d'autres adjuvants compatibles et pharmaceutiquement acceptables. A titre d'exemple elles peuvent également contenir des édulcorants, des arômes, des agents de conservation des colorants ou éventuellement des gélifiants.

Il est entendu que les sels de la sparfloxacine avec le polyhydroxyacide monocarboxylique ainsi obtenus, qui permettent la préparation et l'usage de solutions injectables, stables, entrent aussi dans le cadre de la présente invention.

Les solutions selon l'invention sont préparées alternativement par addition d'eau à un mélange comprenant la sparfloxacine, le/les polyhydroxyacides monocarboxyliques choisis, en quantité au moins stoechiométrique par rapport à la sparfloxacine, le cas échéant l'excès du polyhydroxyacide monocarboxylique ou l'autre acide destiné à assurer un pH de solubilisation du sel de la sparfloxacine inférieur ou égal à 5 et/ou l'isotonisant et les autres adjuvants, ou bien par addition de sparfloxacine et éventuellement des autres additifs à une solution du/des polyhydroxyacides monocarboxyliques.

La préparation et la répartition de la solution sont effectuées de préférence sous azote. Les solutions ainsi obtenues peuvent être stérilisées à la chaleur (stérilisation à l'autoclave). Dans le cas des solutions de sel de l'acide ascorbique, il est néanmoins préférable d'opérer par filtration stérilisante.

Il est également possible de préparer les solutions selon l'invention au moyen de dérivés lactone qui par hydrolyse génèrent in situ les polyhydroxyacides monocarboxyliques.

Les solutions selon l'invention présentent l'avantage d'une très bonne stabilité physico-chimique.

Elles sont particulièrement intéressantes dans la mesure où elles donnent accès à des formulations liquides de la sparfloxacine ce qui jusqu'à présent n'avait pas été réalisable, notamment à une formulation injectable, stable à la lumière dans des conditions normales d'utilisation et stable à la chaleur tout en ayant un pH compatible avec une administration directe. Cette nouvelle formulation est particulièrement avantageuse pour le stockage, la commodité et la rapidité d'utilisation d'une forme prête à l'emploi et aussi dans le cas des perfusions lentes. Par ailleurs, et compte tenu de la bonne solubilité des sels obtenus dans les conditions de pH utilisées, ces solutions peuvent le cas échéant être concentrées, ce qui permet un choix varié des doses à formuler.

Les exemples suivants donnés à titre non limitatif illustrent la présente invention.

### Exemple 1

Préparation d'une solution à 4 mg/ml de sparfloxacine, isotonisée au glucose.

400 mg de sparfloxacine sont mélangés à 321 mg de glucono δ-lactone et 4,75 g de glucose monohydraté. Ce mélange de poudres est dissous dans de l'eau pour préparation injectable préalablement inertée à l'azote. La dissolution est obtenue par agitation sous azote. Le volume est complété à 100 ml avec de l'eau pour préparation injectable. La solution ainsi obtenue est stérilisée à l'autoclave (120°C, 20 minutes).

Le pH de la solution est de 3,8.

Cette solution reste limpide après 5 mois de conservation à 4, 20 et 35°C, sans modification de pH.

### Exemple 2

Préparation d'une solution à 10 mg/ml de sparfloxacine isotonisée au glucose.

En opérant comme précédemment à l'exemple 1, mais en ajustant le volume à 40 ml et en employant 1,74 g de glucose monohydraté, on obtient une solution limpide dont le pH est de 3,8.

Une telle solution préparée à l'échelle de 2,5 litres reste limpide et n'est pas modifiée en pH sur une période d'au moins 5 mois et dans les conditions de température suivantes : 20°C, 4°C, et 35°C.

Une analyse HPLC effectuée après 3 mois de conservation à 20°C, 4°C 35°C et 45°C n'a pas montré de dégradation du produit.

### Exemple 3

Préparation d'une solution à 20 mg/ml de sparfloxacine isotonisée au glucose.

En opérant comme précédemment à l'exemple 1 mais en ajustant le volume à 20 ml et par addition de 770 mg de glucose monohydraté, on prépare une solution limpide dont le pH est 3,7.

Cette solution préparée à l'échelle de 2 litres reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 4

Préparation d'une solution à 50 mg/ml de sparfloxacine isotonisée au glucose.

En opérant comme à l'exemple 1, mais en ajustant le volume à 8 ml et par addition de 130 mg de glucose monohydraté, on prépare une solution limpide dont le pH est 3,5.

Cette solution préparée à l'échelle de 2 litres reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 5

Préparation d'une solution à 100 mg/ml de sparfloxacine, hypertonique.

En opérant comme à l'exemple 1, mais en ajustant le volume à 4 ml et sans addition de glucose, on prépare une solution dont le pH est de 3,5.

Cette solution préparée à l'échelle de 1 litre reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 6

Préparation d'une solution à 200 mg/ml de sparfloxacine, hypertonique.

En opérant comme à l'exemple 1, mais en ajustant le volume à 2 ml et sans ajouter de glucose, on prépare une solution limpide dont le pH est 3,5.

Cette solution préparée à l'échelle de 1 litre reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 7

Préparation d'une solution à 10 mg/ml de sparfloxacine.

1000 mg de sparfloxacine sont mélangés à 1,79 g d'acide lactobionique. Ce mélange de poudres est dissous dans de l'eau pour préparation injectable préalablement inertée à l'azote. La dissolution est obtenue par agitation sous azote. Le volume est complété à 100 ml avec de l'eau pour préparation injectable. La solution ainsi obtenue est stérilisée à l'autoclave (120°C, 20 minutes).

Le pH de la solution est de 3,75.

Cette solution conservée 5 mois à 4 et à 20°C reste limpide et sans modification de pH.

Un dosage par HPLC après 5 mois de conservation ne montre pas d'impureté de dégradation.

### Exemple 8

Préparation d'une solution à 20 mg/ml de sparfloxacine.

En opérant comme à l'exemple 7, mais à partir de 2000 mg de sparfloxacine et de 3,58 g d'acide lactobionique et en ajustant le volume à 100 ml, on obtient une solution limpide de pH = 3,5.

### Exemple 9

Préparation d'une solution à 30 mg/ml de sparfloxacine.

En opérant comme à l'exemple 7, mais à partir de 3000 mg de sparfloxacine et de 8,96 g d'acide lactobionique et en ajustant le volume à 100 ml, on obtient une solution limpide de pH = 3,5.

### Exemple 10

Préparation d'une solution à 10 mg/ml de sparfloxacine.

En opérant comme à l'exemple 7, mais à partir de 1000 mg de sparfloxacine et de 2,26 g d'acide glucoheptonique et en ajustant le volume à 100 ml, après chauffage à 50°C pendant 1 heure, on obtient une solution limpide de pH = 4,3.

### Exemple 11

Préparation d'une solution à 10 mg/ml de sparfloxacine isotonisée au glucose.

1000 mg de sparfloxacine sont mélangés à 528 mg d'acide ascorbique et 4 g de glucose anhydre. Ce mélange de poudres est dissous dans de l'eau pour préparation injectable préalablement inertée à l'azote. La dissolution est obtenue par agitation sous azote. Le volume est complété à 100 ml avec de l'eau pour préparation injectable. La solution ainsi obtenue est stérilisée par filtration stérilisante.

Le pH de la solution est de 4,8.

Cette solution conservée 7 mois à 4 et à 20°C, à l'abri de la lumière, reste limpide et sans modification de pH.

Un dosage par HPLC après 7 mois de conservation ne montre pas d'impureté de dégradation.

### Exemple 12

Préparation d'une solution à 10 mg/ml de sparfloxacine, isotonisée au glycérol.

En opérant comme à l'exemple 11, mais à partir de 2,2 g de glycérol au lieu du glucose, on obtient une solution limpide de pH = 5.

Cette solution reste limpide après 7 mois à 4 et 20°C, à l'abri de la lumière et sans modification de pH.

Un dosage par HPLC après 7 mois de conservation ne montre pas d'impureté de dégradation.

### Exemple 13

Préparation d'une solution à 20 mg/ml de sparfloxacine.

En opérant comme à l'exemple 11, mais à partir de 2000 mg de sparfloxacine et de 1,76 g d'acide ascorbique, on obtient une solution limpide de pH = 4,2.

Cette solution reste limpide après 7 mois à 20°C, à l'abri de la lumière.

## Revendications

1. Solution aqueuse stable de la sparfloxacine de formule : 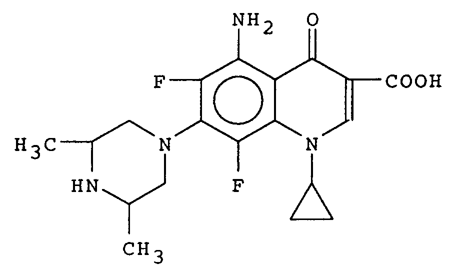 caractérisée en ce qu'elle contient :
- la sparfloxacine,
- un ou plusieurs polyhydroxyacides monocarboxyliques choisis parmi les acides ayant un pK_{A} supérieur à 3 à 25°C ou leurs dérivés lactone, au moins en quantité stoechiométrique par rapport à la sparfloxacine,
- si nécessaire un excès du polyhydroxyacide monocarboxylique ou d'un autre acide pharmaceutiquement acceptable destiné à assurer un pH de solubilisation complète du sel ainsi formé, inférieur ou égal à 5,
- éventuellement un agent isotonisant et/ou d'autres adjuvants pharmaceutiquement acceptables.

2. Une solution selon la revendication 1 ou 2, caractérisée en ce que le polyhydroxyacide monocarboxylique est choisi parmi :
- l'acide lactobionique,
- l'acide glucoheptonique,
- l'acide gluconique ou
- l'acide ascorbique.

3. Une solution selon la revendication 1, caractérisée en ce qu'elle contient une concentration allant jusqu'à 40 % de sparfloxacine.

4. Une solution selon la revendication 1, caractérisée en ce que son pH est compris entre 3,5 et 5.

5. Une solution selon la revendication 1, caractérisée en ce que l'isotonisant est choisi parmi le glucose, le glycérol, le sorbitol, le mannitol, le xylitol, le fructose, ou le lactose.

6. Préparation d'une solution selon la revendication 1, caractérisée en ce qu'un mélange de sparfloxacine, d'un ou plusieurs polyhydroxyacides monocarboxyliques en quantité au moins stoechiométrique par rapport à la sparfloxacine et éventuellement d'un excès du polyhydroxyacide monocarboxylique ou d'un autre acide capable d'assurer un pH de solubilisation complète du sel inférieur ou égal à 5 et/ou d'un isotonisant et d'autres adjuvants est additionné d'eau.

7. Préparation d'une solution selon la revendication 1, caractérisée en ce que la sparfloxacine, éventuellement un acide capable d'assurer un pH de solubilisation complète du sel inférieur ou égal à 5 et/ou un isotonisant et d'autres adjuvants sont ajoutés à une solution d'un ou plusieurs polyhydroxyacides monocarboxyliques.

8. Un sel de la sparfloxacine avec un polyhydroxyacide monocarboxylique choisi parmi :
- l'acide lactobionique,
- l'acide glucoheptonique,
- l'acide ascorbique.

9. Composition pharmaceutique caractérisée en ce qu'elle contient une solution selon la revendication 1, éventuellement en association avec un ou plusieurs diluants ou adjuvants compatibles et pharmaceutiquement acceptables.

## Claims

1. Stable aqueous solution of sparfloxacin of formula: 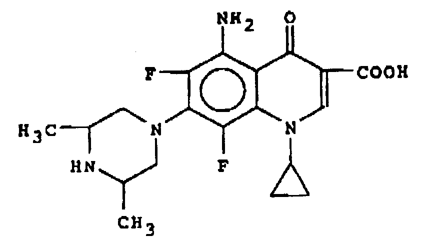 characterized in that it contains:
- sparfloxacin,
- one or more polyhydroxymonocarboxylic acids chosen from amongst the acids having a pK_{A} greater than 3 at 25°C or their lactone derivatives, at least in a stoichiometric quantity with respect to sparfloxacin,
- if necessary an excess of the polyhydroxymonocarboxylic acid or of another pharmaceutically acceptable acid intended to ensure a pH of complete solubilization of the salt thus formed, of less than or equal to 5,
- if appropriate, an isotonizing agent and/or other pharmaceutically acceptable adjuvants.

2. Solution according to Claim 1 or 2, characterized in that the polyhydroxymonocarboxylic acid is chosen from amongst:
- lactobionic acid,
- glucoheptonic acid,
- gluconic acid or
- ascorbic acid.

3. Solution according to Claim 1, characterized in that it contains a concentration increasing to 40 % of sparfloxacin.

4. Solution according to Claim 1, characterized in that its pH is between 3.5 and 5.

5. Solution according to Claim 1, characterized in that the isotonizing agent is chosen from amongst glucose, glycerol, sorbitol, mannitol, xylitol, fructose or lactose.

6. Preparation of a solution according to Claim 1, characterized in that water is added to a mixture of sparfloxacin, one or more polyhydroxymonocarboxylic acids in an at least stoichiometric quantity with respect to sparfloxacin and, if appropriate, an excess of the polyhydroxymonocarboxylic acid or of another acid capable of ensuring a pH of complete solubilization of the salt of less than or equal to 5 and/or a tonicity regulator and other adjuvants.

7. Preparation of a solution according to Claim 1, characterized in that the sparfloxacin, if appropriate, an acid able to ensure a complete solubilization pH of the salt of less than or equal to 5 and/or a tonicity regulator and other adjuvants are added to a solution of one or more polyhydroxymonocarboxylic acids.

8. Salt of sparfloxacin with a polyhydroxymonocarboxylic acid chosen from amongst:
- lactobionic acid,
- glucoheptonic acid,
- ascorbic acid.

9. Pharmaceutical composition characterized in that it contains a solution according to Claim 1, if appropriate in association with one or more compatible and pharmaceutically acceptable diluents or adjuvants.

## Patentansprüche

1. Stabile wäßrige Lösung von Sparfloxazin der Formel: 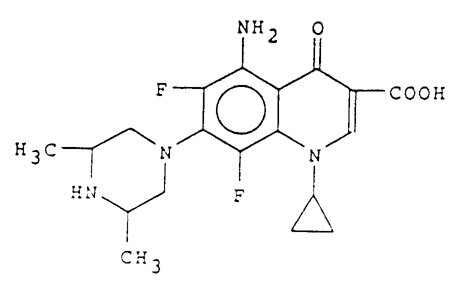 dadurch **gekennzeichnet,** daß sie enthält:
- Sparfloxazin,
- eine oder mehrere Polyhydroxymonocarbonsäure(n), ausgewählt aus Säuren mit einem pK_{A}-Wert von größer 3 bei 25°C, oder ihre Lactonderivate, mindestens in stöchiometrischer Menge, bezogen auf Sparfloxazin,
- sofern notwendig, einen Überschuß an Polyhydroxymonocarbonsäure oder einer anderen pharmazeutisch verträglichen Säure zur Gewährleistung eines pH-Werts der vollständigen Solubilisation des so gebildeten Salzes von kleiner oder gleich 5,
- gegebenenfalls ein Isotonisierungsmittel und/oder andere pharmazeutisch verträgliche Adjuvantien.

2. Lösung nach Anspruch 1, dadurch **gekennzeichnet,** daß die Polyhydroxymonocarbonsäure ausgewählt ist aus:
- Lactobionsäure,
- Glucoheptonsäure
- Gluconsäure oder
- Ascorbinsäure.

3. Lösung nach Anspruch 1, dadurch **gekennzeichnet,** daß sie eine Konzentration bis zu 40% Sparfloxazin enthält.

4. Lösung nach Anspruch 1, dadurch **gekennzeichnet,** daß ihr pH-Wert zwischen 3,5 und 5 liegt.

5. Lösung nach Anspruch 1, dadurch **gekennzeichnet,** daß das Isotonisierungsmittel aus Glucose, Glycerin, Sorbit, Mannit, Xylit, Fructose oder Lactose ausgewählt ist.

6. Verfahren zur Herstellung einer Lösung gemäß Anspruch 1, dadurch **gekennzeichnet,** daß man ein Gemisch aus Sparfloxazin, einer oder mehreren Polyhydroxymonocarbonsäure(n), in mindestens stöchiometrischer Menge, bezogen auf das Sparfloxazin, und gegebenenfalls eines Überschusses an Polyhydroxymonocarbonsäure oder einer anderen Säure mit der Fähigkeit zur Gewährleistung eines pH-Werts zur vollständigen Solubilisation des Salzes von kleiner oder gleich 5 und/oder eines Isotonisierungsmittels und anderer Adjuvantien mit Wasser versetzt.

7. Verfahren zur Herstellung einer Lösung gemäß Anspruch 1, dadurch **gekennzeichnet,** daß man Sparfloxazin, gegebenenfalls eine Säure mit der Fähigkeit, einen pH-Wert zur vollständigen Solubilisation des Salzes von kleiner oder gleich 5 zu gewährleisten und/oder ein Isotonisierungsmittel und andere Adjuvantien einer Lösung aus einer oder mehreren Polyhydroxymonocarbonsäure(n) zusetzt.

8. Salz aus Sparfloxazin mit einer Polyhydroxymonocarbonsäure, ausgewählt aus:
- Lactobionsäure,
- Glucoheptonsäure,
- Ascorbinsäure.

9. Pharmazeutische Zusammensetzung, dadurch **gekennzeichnet,** daß sie eine Lösung nach Anspruch 1, gegebenenfalls zusammen mit einem oder mehreren Verdünnungsmittel(n) oder kompatiblen und pharmazeutisch verträglichen Adjuvantien enthält.
